(19) Europäisches Patentamt / European Patent Office / Office européen des brevets

(11) **EP 1 639 120 B1**

(12) **EUROPEAN PATENT SPECIFICATION**

(45) Date of publication and mention
of the grant of the patent:
**02.05.2007 Bulletin 2007/18**

(51) Int Cl.:
*C12P 7/64* (2006.01)   *C11B 3/00* (2006.01)
*C11B 3/10* (2006.01)

(21) Application number: **04739748.4**

(22) Date of filing: **09.06.2004**

(86) International application number:
**PCT/EP2004/006243**

(87) International publication number:
**WO 2005/003365 (13.01.2005 Gazette 2005/02)**

(54) **PROCESS FOR INTERESTERIFICATION OF A GLYCERIDE FAT**

VERFAHREN ZUR INTERESTERIFIKATION VON GLYCERIDEFETT

PROCEDE D'INTERESTERIFICATION D'UNE GRAISSE GLYCERIDE

(84) Designated Contracting States:
**AT BE BG CH CY CZ DE DK EE ES FI FR GB GR HU IE IT LI LU MC NL PL PT RO SE SI SK TR**

(30) Priority: **27.06.2003 EP 03077099**

(43) Date of publication of application:
**29.03.2006 Bulletin 2006/13**

(73) Proprietors:
• **UNILEVER N.V.**
**3013 AL Rotterdam (NL)**
Designated Contracting States:
**AT BE BG CH CZ DE DK EE ES FI FR GR HU IT LI LU MC NL PL PT RO SE SI SK TR**
• **UNILEVER PLC**
**London, Greater London EC4P 4BQ (GB)**
Designated Contracting States:
**CY GB IE**

(72) Inventors:
• **TEN BRINK, Hilda B.,**
**Unilever R & D Vlaardingen**
**3133 AT Vlaardingen (NL)**
• **DIENDER, M.B.,**
**Unilever Bestfoods North America**
**Baltimore,**
**Maryland 21229 (US)**
• **DIKS, Robertus M.,**
**Unilever R & D Vlaardingen**
**3133 AT Vlaardingen (NL)**
• **KRAMER, Gerard**
**3111 PX Schiedam (NL)**
• **MUIS, Leo,**
**Unilever R & D Vlaardingen**
**3133 AT Vlaardingen (NL)**

• **POTMAN, Ronald, Peter,2Unilever R & D Vlaardingen**
**3133 AT Vlaardingen (NL)**

(74) Representative: **Wurfbain, Gilles L. et al**
**Unilever Patent Group**
**Olivier van Noortlaan 120**
**3133 AT Vlaardingen (NL)**

(56) References cited:
**WO-A-02/081719          US-B1- 6 398 707**

• **TORRES C F ET AL: "CATALYTIC TRANSESTERIFICATION OF CORN OIL AND TRISTEARIN USING IMMOBILIZED LIPASES FROM THERMOMYCES LANUGINOSA" JOURNAL OF THE AMERICAN OIL CHEMISTS' SOCIETY, AMERICAN OIL CHEMISTS' SOCIETY. CHAMPAIGN, US, vol. 79, no. 8, August 2002 (2002-08), pages 775-781, XP001124617 ISSN: 0003-021X**
• **LIE E., ET AL: "Hydrolysis and esterification with immobilized lipase on hydrophobic and hydrophilic zeolites" JOURNAL OF CHEMICAL TECHNOLOGY AND BIOTECHNOLOGY. (INTERNATIONAL JOURNAL OF BIOTECHNICAL AND CHEMICAL PROCESSES), ELSEVIER APPLIED SCIENCE PUBLISHERS. BARKING, GB, vol. 50, 1991, pages 549-553, XP002240666 ISSN: 0268-2575**
• **SHARMA R ET AL: "Production, purification, characterization, and applications of lipases" BIOTECHNOLOGY ADVANCES, ELSEVIER PUBLISHING, BARKING, GB, vol. 19, no. 8, December 2001 (2001-12), pages 627-662, XP004343846 ISSN: 0734-9750**

**Description**

The present invention deals with a process for modification of glyceride fats which process is denoted as interesterification and particularly deals with enzymatic interesterification.

**BACKGROUND AND PRIOR ART**

Glyceride fats are major constituents of many food products. For nutritional and economic reasons use of vegetable fats is preferred. The major part of fats consist of triglyceride molecules, three fatty acid residues esterified to a glycerol backbone. Often fats contain small, variable amounts of partial glycerides. Partial glycerides occur as natural constituents of vegetable fats. They may be formed by hydrolysis of one or two fatty acid residues on the glycerol backbone. Natural fats are a blend of many kinds of triglycerides. Triglycerides and partial glycerides are present in fats in amounts which vary with the nature and the origin of the fat. Most vegetable fats are liquid at ambient temperature and then they are denoted as oils. From a chemical point of view there is no difference between fats and oils and the terms are used interchangeably. Natural vegetable fats often need some modification for making them suited as a food ingredient. Modification processes comprise blending with another fat, hydrogenation, fractionation and interesterification.

The present invention deals with fat modification by interesterification. Interesterification aims at rearranging the fatty acid residues over the positions on the glyceride backbone of the present fat molecules with the effect that new types of triglycerides and partial glycerides are formed and the amounts of the various glycerides get changed. An interesterification process usually is carried out in the presence of an interesterification catalyst. The aimed redistribution or rearrangement of fatty acid residues proceeds random over all three positions when a chemical catalyst is used. But, generally, rearrangement on the terminal positions (1,3) only occurs when an enzyme is used as catalyst. Several lipase enzymes have been found to have the functionality to act as interesterification catalyst. Some of these are commercially available.

Enzymatic interesterification, also denoted as enzymatic rearrangement, proceeds as an equilibrium reaction in which in one direction on the one side triglycerides are hydrolysed to free fatty acids and glycerol, mono- and/or diglycerides, and in the opposite direction glycerol, monoglycerides and diglycerides are re-esterified to triglycerides and partial glycerides. While chemical interesterification proceeds nearly instantaneous, enzymatic rearrangement proceeds gradually starting from 0% and finally attains 100% rearrangement, which may take days or weeks depending on the quality of the enzyme catalyst and the rate of its deactivation. 100% rearrangement means that a stable equilibrium composition of glyceride molecules is attained.
The lower the degree of rearrangement the shorter the necessary time of exposure of oil to lipase catalyst. So it is possible to produce in one day more partially rearranged oil than fully rearranged oil, using identical batches of lipase catalyst.

Literature on enzymatic rearrangement of triglyceride fats is abundant. The process is described in general terms in The Lipid Handbook (Ed. F.D. Gunstone et al., first ed. 1986, page 478).

Preferably a lipase enzyme is used for enzymatic rearrangement after being immobilized on a carrier substance. As carrier substance, generally, fat insoluble porous particulate materials are used which provide a high surface area per unit volume. Immobilized enzymes may act while dispersed in the oil to be modified, but a rearrangement process preferably employs a packed bed reactor, which is a container provided with an inlet and an outlet for an oil flow and which is filled with a packed bed of immobilized enzyme particles allowing the oil to be modified while being conducted through the bed of lipase catalyst. In this specification the conglomerate of lipase and carrier substance is denoted lipase catalyst.

The use of lipase enzyme as interesterification catalyst is associated with a number of problems. A major one is the short lifetime of the lipase enzyme. A short lifetime means that when the reaction proceeds the enzyme's catalytic activity quickly drops. In order to get the oil sufficiently rearranged the time of contacting oil with catalyst has to be prolonged continuously, finally beyond a point that the process is no longer economically feasible and replacement of the catalyst becomes necessary. Lifetime is more precisely expressed as halflife time, the process time in which half of the activity of the employed enzyme is lost. Short halflife times result in a low productivity values. Frequent refreshment of the lipase is a less desired option, because of the high costs of the enzyme.

It is known since long that pre-treating the substrate oil with an adsorbing substance before exposing the oil to the enzyme prolonges lifetime of the enzyme and so improves the poor economy of the process. JP 08000275 teaches that stirring the oil at 110°C for 20 minutes with 2% acidic clay, e.g. Supreme™ which is a clay activated by a treatment with sulphuric acid, increases the halflife of the enzyme from 43 hours to more than 150 hours.

WO 02/081719 discloses an alternative proposal for prolonging enzyme halflife by employing silica adsorbent for a purifying pre-treatment.

The productivity improvements by prolonging halflife always have been realised with small scale laboratory processes only. In the prior art no mention can be found of a commercial enzymatic interesterification processes which is applied on an industrial scale. In order to get an enzymatic rearrangement process economically feasible on an industrial scale it has to proceed with a throughput which must be at least 4000 kg of rearranged oil in 2 days necessitating large process vessels either in batch mode or in continuous mode using a packed bed reactor. However, no industrial scale manufacturing plants have been built, although since long an ever increasing need exists for substituting chemical interesterification by an enzymatic, hence natural process. Satisfactory laboratory scale enzymatic interesterification processes always failed when upgraded to industrial scale. We have found that the pre-treatment of the substrate oil with an adsorbing substance, although effective in the laboratory, fails when the treatment is carried out at an industrial scale. See the data of Table I in the example. Our aim was to create an industrial scale process for enzymatic rearrangement which is economically competitive with present chemical interesterification processes.

## SUMMARY OF THE INVENTION

The present invention comprises an oil modification process which comprises exposing a glyceride oil to a lipase catalyst with the effect of rearrangement of fatty acid residues on the glyceride molecules, which rearrangement is carried out to a degree of 5 - 99%, using a lipase catalyst having a productivity value (LP) > 2500 - which means that within a standard period of three weeks at least 2500 kg of glyceride oil is enzymatically rearranged to a standard degree of 95% with a standard consumption of 1 kg of lipase catalyst - which process is characterized in that it proceeds with an industrial throughput of at least 4000 kg oil in the first 48 hours; and wherein the oil in a pretreatment is exposed to an adsorbent which is dispersed in the oil to form an oil/adsorbant dispersion, and said adsorbent is removed before the oil is brought in contact with the lipase catalyst, and wherein the oil/adsorbent dispersion is exposed to shear energy dissipated in the dispersion which exceeds 0.5 W/kg, preferably exceeds 0.75 W/kg, more preferably exceeds 1 W/kg and still more preferably exceeds 2.5 W/kg.

The invention provides the conditions under which the process is able to proceed with said high catalyst productivity.

## DETAILS OF THE INVENTION

The aim of the invention is pursued by prolonging halflife of the enzyme when employed for an industrial scale process, with the effect that the lipase catalyst productivity is increased.

An industrial enzymatic rearrangement process employs a reactor which contains a packed bed of enzyme catalyst. Starting with fresh catalyst the initial oil flow is high, but has to be reduced gradually in order to keep pace with the gradually decreasing catalyst activity. Otherwise the rearrangement degree would drop below the set value. The process of the patent is characterised by a throughput of at least 4000 kg oil which results from the first 48 hours of enzyme/oil contact.

We also have found conditions which enable the claimed process to proceed with a lipase catalyst productivity (LP) which is > 2500, preferably is > 4000 and more preferably is > 5500. The LP value is calculated using a protocol as described later in this specification.

These high productivity levels are unique for a process with a throughput in the first 48 hours of at least 4000 kg and preferably of at least 15000 kg, more preferably of at least 30000 kg, still more preferably at least 45000 kg and still more preferably at least 90000 kg.

As mentioned above prior art references teach the use of an adsorbing substance for a purifying pretreatment of the oil to be modified. In WO 02/081719 a large group of known adsorbing substances has been listed. However, when using the sole exemplified adsorbent, silica, the resulting extension of the enzyme halflife was not sufficient. It was unexpectedly found that the factor determining effective removal of catalyst poisons from the oil is maintenance of a thorough contact of oil and adsorbent. While this thorough shear contact, which is crucial for effective removal of enzyme poisons, can be easily realised in a laboratory vessel by normal stirring of the dispersion of oil and adsorbent, it has been found necessary to take unusual measures to ensure the same shear conditions in the large vessels which are necessary when initial throughputs of at least 4000 kg oil have to be realized. For that aim highly effective stirrers with a shear energy output exceeding 0.5 W/kg are required. Alternatively an effective shear regime can be realized by forceful recirculation using a gear pump. Also dosing the adsorbent in the oil through a venturi pipe or by passing a colloid mill may realize the effective shear contact of oil and adsorbent.

Preferably, the amount of shear energy dissipated in the oil/adsorbent dispersion to which the mixture is exposed exceeds 0.75 W/kg, more preferably exceeds 1 W/kg and still more preferably exceeds 2.5 W/kg.

The oil is kept in contact with the adsorbent preferably for a period of 5 minutes to 12 hours and more preferably for a period of 1 - 6 hours. The contact time has an optimum which is less than 4 hours. In order to prevent clogging of the packed bed reactor, the adsorbent is removed before the oil is exposed to the lipase catalyst, preferably with the help of a filtration aid, such as celite.

When the fat to be modified is not liquid, it should be submitted to the process of the invention at a temperature at which it has become liquefied.

During exposure to the adsorbent the oil has a temperature preferably being in the range 30 - 150°C, more preferably in the range 40 - 110°C and still more preferably in the range 70 - 90°C.

Preferably, the oil before its exposure to the adsorbent is treated with bleaching earth at a temperature selected from the range 85 - 220°C, preferably 85 - 120°C. The adsorbent may be added to the oil either when it still has to be submitted to the bleaching treatment or after such treatment.

Zeolites were found to show unexpectedly high effectivity, provided the substrate oil was pretreated with the adsorbent under proper shear conditions.

The zeolite should be a basic zeolite and is applied in an amount which preferably is chosen from the range 0.01 - 10 wt.% on oil, preferably from the range 0.1 - 0.5 wt.%. Provided thorough shear contact is ensured, already small amounts of zeolite have appeared to be effective.

Zeolites belong to a class of highly structured alumina silicates which counts many representatives. Most zeolites are made artificially, but some are found as minerals in nature, such as mordenite and clinoptilolite. A major commercial application of a zeolite is its use as a washing composition ingredient for softening water.

The porous structure of zeolite consists of a crystal lattice in which oxygen, silicon and aluminium atoms are placed in such array that they can easily harbour water molecules and also host various cations which further determines the zeolite's functionality, particularly the adsorption of undesired substances.

Commercial, synthetic zeolites are categorized, depending on structure and functionality, as P-, A-, X- and Y-zeolites, with the general formula 0.8-1.5 $Na_2O$. $Al_2O_3$. 0.8-24 $SiO_2$, where Si:Al = 1 for A- and P-zeolites and Si:Al = 1 - 6 for X- and Y-zeolites.

When used in a pretreatment the purifying activity of different zeolites may vary, but effective zeolites are selected preferably from the P-, A-, X- and Y-zeolites. Good representatives of commercially available zeolites can be found in the group consisting of Doucil A24™, Doucil 4A™ (Wessalyth P) and CBV 100™ and in mixtures of two or more of these.

For each type of substrate oil the optimum conditions for a pretreatment with an adsorbent can be easily found by some trial experiments.

The subsequent rearrangement process may be carried out in batch mode by stirring the oil with the added lipase catalyst in a vessel provided with a heating system, but the preferred mode is the usual continuous process which comprises conducting the oil through a reactor with a packed catalyst bed.

The commercially available lipases are suitably obtained by culturing specific microorganisms such as Rhizomucor miehei. Preferably, a commercial lipase catalyst is selected from the group consisting of Lipase D™ (ex Amano), Lipozyme TL IM™ (ex Novozymes) and Lipozyme RM IM™ (ex Novozymes) and mixtures of two or more of these. More preferably the lipase catalyst contains Thermomyces lanuginosa lipase enzyme.

The lipase preferably is employed immobilised on a suitable carrier substance, which preferably is chosen from the group consisting of polypropylene, e.g. Accurel™ (ex ACCORDIS MEMBRANES GmbH) and silica or a mixture of these.

The oil is exposed to the lipase at a temperature preferably being in the range 30 - 75°C, and preferably in the range 60 - 70°C.

The process of the invention can be generally applied and is suitable for processing various types of oil. A single oil may be processed or a blend of several oils. Good results have been obtained with traditional blends like mixtures of palm kernel oil with palm oil fractions and of soybean oil with fully hardened soybean oil. Palmkernel oil often is substituted by coconut oil.

It is immaterial for the process whether the glyceride fat to be modified contains besides triglycerides also partial glycerides, even substantial amounts. These also become rearranged.

For the enzymatic rearrangement standard process conditions as known to the skilled man can be used or as advised by the supplier of the enzyme catalyst.

The invention teaches the observation of a proper shear regime during pretreatment of the substrate oil. The result being an effective prolongation of enzyme halflife turns enzymatic interesterification into an economically feasible fat modification process.

The following example illustrates the invention.

**PROTOCOL FOR MEASURING LIPASE CATALYST PRODUCTIVITY (LP)**

The LP value is calculated by first establishing the amount of oil which is rearranged to a standard degree of 95% and the amount of catalyst which has been consumed in that process. Catalyst is considered to be consumed when its activity has dropped to less than 10% of its initial activity. Any standard lipase activity measurement is suited. The LP value is found by dividing the amount of oil by the amount of consumed catalyst. The amount of catalyst should be chosen such that the catalyst is just consumed after three weeks of process time. When the moment of exhaustion is outside the three weeks period, the LP value is estimated by extrapolation to three weeks.

**PROTOCOL FOR MEASURING DEGREE OF REARRANGEMENT**

The degree of rearrangement at any stage of the proceeding reaction is determined as follows:

For any starting fat blend (the feedstock) and for an actual reaction product the overall fatty acids composition and the triglyceride composition is established using common analysis methods comprising FAME-analysis, the GLC/carbon number method and the HPLC/silver phase method as are described in for example EP 78568, EP 652289, JAOCS, (1991), 68(5), 289-293 and Hammond E.W.J., Chromatography, 203, 397, 1981.

Determination of the degree of rearrangement for the enzymatically catalyzed reaction is based on the change of the carbon number profile. For a specific reaction product and its starting material the carbon number profile is determined by silverphase chromatography following the prior art methods as described above. The carbon number is the total number of carbon atoms in the three fatty acid residues of a triglyceride molecule.

The carbon number profile of a fully randomized product is a theoretical value calculated from the measured triacylglyceride profile of the starting mixture. Experimental information necessary to perform this calculation can be obtained by analyzing the profile of fatty acid residues of the fat composition using the above mentioned methods.

From the resulting triacylglycerol profile the carbon number profile can easily be derived.

The degree of rearrangement is calculated according to the following scheme:

For each carbon number in the range 30 - 60 the differences are calculated between the mole fraction at the begin of the reaction and at the 100% randomisation state. The sum of the absolute values of these differences defines the 100% absolute change of carbon numbers which points to the 100% rearrangement state.

A specific sample is taken from the proceeding reaction and in the same way for each carbon number between 30 to 60 the differences between the fraction at the begin of the reaction and the fraction at the actual state of the reaction product are calculated. Again the sum of the absolute value of these differences is taken. This number is the actual absolute change of carbon numbers.

```
The degree of actual rearrangement = (actual absolute
change of carbon numbers) / (100% absolute change of carbon
numbers).
```

This equation applies for triglyceride products where the 100% absolute change of carbon numbers is at least 0.15. If not, the degree of rearrangement is determined in an alternative way:

The mole fractions are established for each triacylglyceride of the type H3, H2O and H2L in the starting blend, where H indicates fatty acid residues of palmitic or stearic acid, O of oleic acid and L of linoleic acic. H3 denotes a triacylglyceride containing 3 H type fatty acid residues and so forth for H2O and H2L. For each of these triglycerides the absolute change between the molar fraction at the begin of the reaction and at the 100% rearrangement state is calculated. The sum of the absolute values of these changes defines the 100% degree of rearrangement, the 100% absolute change of triacylglyceride groups.

For a specific sample taken from the proceeding reaction the differences between the mole fraction at the begin of the reaction and at the actual state of the reaction product is calculated for the same set of triglycerides. The sum of the absolute values of these changes is taken. This number is the actual absolute change of triacylglyceride groups.

For the specific sample the degree of rearrangement = (actual absolute change of triacylglyceride groups) / (100% absolute change of triacylglyceride groups).

**Example 1**

A mixture of 62 wt.parts of a dry fractionated palm oil stearin (slip m.p. 56°C) and 38 wt.parts of palm kernel oil was subjected to a standard bleaching treatment. After removal of the bleaching earth zeolite (Doucil A24™, ex INEOS SILICAS The Netherlands) was added and the mixture was vigorously stirred for one hour at 70°C.

The zeolite treatment was carried out at laboratory scale (10 1 batch), pilot plant scale (20 1 batch) and factory scale (48000 1 batch). Process conditions can be found in Table I.

The shear energy necessary for the pretreatment of the five batches was dissipated by blade stirrers (laboratory and pilot plant batches 1, 2 and 3), a large gate stirrer (factory batch 4) and by recirculation employing a gear pump (factory batch 5). The table mentions the various shear energies dissipated in the five pretreatment batches.

After filtration of the zeolite the purified oil was exposed to immobilized Lipase TL (ex NOVOZYMES) at a temperature of about 70°C. The amount of enzyme and the initial oil flow were such that 95% rearrangement was attained at the end of the exposure.

In order to prevent dropping the reaction rate too much during the subsequent rearrangement process, it was necessary to compensate for the gradual enzyme deactivation by reducing the flow. It appeared that enzyme deactivation was different depending on the scale on which pretreatment of the crude oil had been carried out.

When working in batch mode deactivation could be compensated by regularly adding fresh enzyme.

The productivity LP of the lipase catalyst was calculated according to the above protocol. The values for batches 1 - 5 are shown in Table I.

It has appeared that an increase of the amount of zeolite from 0.05 wt.% to 0.4 wt.% has not any further improving effect on catalyst productivity (see Table I, batches 1 and 2). However, the amount of shear energy dissipated in the pretreatment appears to be a quite critical factor. A shear input below 0.5 W per kg oil is not able to activate sufficient clearing functionality of the present zeolite.

When working at industrial scale normal stirring of the pretreatment dispersion of oil and adsorbent is not good enough.

### TABLE I

| Enzymatic rearrangement at 70°C of an oil blend (2) using Lipozyme TL IM™ (3) | | | | | |
|---|---|---|---|---|---|
| Batch | Zeolite (4) wt.% | Treatment Shear energy / kg oil | Temp/Time | Process Scale | LP (1) |
| 1 | 0.05% | Blade stirrer, 10 W/kg | 65°, 1h | "Lab", 10 l | 6000 |
| 2 | 0.4% | Blade stirrer, 10 W/kg | 65°, 1h | "Lab", 10 l | 6000 |
| 3 | 0.1% | Blade stirrer, 34 W/kg | 70°, 1h | "Pilot plant", 20 l | 8000 |
| 4 | 0.1% | Gate stirrer, 0.36 W/kg | 70°, 1h | "Factory", 48000 l | 1500 |
| 5 | 0.3% | Recirculation pump, 0.75 W/kg | 70°, 1h | "Factory", 48000 l | 6000 |
| (1) LP = lipase catalyst productivity: the amount of glyceride oil which within a standard period of three weeks is enzymatically rearranged to a standard degree of 95% with a standard consumption of 1 kg of lipase catalyst.<br>(2) A mixture of 62 wt.parts dry fractionated palm oil stearin (slip mp. 56°C) and 38 wt.parts palm kernel oil.<br>(3) Thermomyces lanuginosa immobilized on silica (ex NOVOZYMES DK).<br>(4) Doucil A24™. | | | | | |

## Claims

1. An oil modification process which comprises exposing a glyceride oil to a lipase catalyst with the effect of rearrangement of fatty acid residues on the glyceride molecules, which rearrangement is carried out to a degree of 5 - 99%, using a lipase catalyst having a productivity value (LP) > 2500 - which means that within a period of three weeks at least 2500 kg of glyceride oil is enzymatically rearranged to a degree of 95% with a consumption of 1 kg of lipase catalyst-which process proceeds with an industrial throughput of at least 4000 kg oil in the first 48 hours, and wherein the oil in a pretreatment is exposed to an adsorbent which is dispersed in the oil to form an oil/adsorbant dispersion, and said adsorbent is removed before the oil is brought in contact with the lipase catalyst, and wherein the oil/adsorbent dispersion is exposed to shear energy dissipated in the dispersion which exceeds 0.5 W/kg, preferably exceeds 0.75 W/kg, more preferably exceeds 1 W/kg and still more preferably exceeds 2.5 W/kg.

2. A process according to claim 1, **characterized in that** the lipase of the lipase catalyst is immobilized on a carrier substance, preferably on polypropylene or silica or a mixture of these.

3. A process according to claim 1, **characterized in that** the lipase catalyst contains Thermomyces lanuginosa lipase enzyme.

4. A process according to claim 1, **characterized by** the use of a packed bed reactor.

5. A process according to claim 1, **characterized in that** during exposure to the lipase the oil has a temperature being in the range 30 - 75°C, preferably being in the range 60 - 70°C.

6.  A process according to claim 1, **characterized in that** the oil is in contact with the adsorbent for a period chosen from the range 5 minutes to 12 hours and preferably from the range 1 to 6 hours.

7.  A process according to claim 1, **characterized in that** during exposure to the adsorbent the oil has a temperature being in the range 30 - 150°C, preferably in the range 40 - 110°C and more preferably in the range 70 - 90°C.

8.  A process according to claim 1, **characterized in that** the oil before exposure to the adsorbent is submitted to a usual bleaching treatment at a temperature selected from the range 85 - 220°C, preferably from the range 85 - 120°C.

9.  A process according to claim 1, **characterized in that** the adsorbent is removed from the oil with the help of a filtration aid, which preferably is celite.

10. A process according to claim 1, **characterized in that** the adsorbent is a zeolite.

11. A process according to claim 10, **characterized in that** the amount of zeolite is chosen from the range 0.01 - 10 wt.% on oil, preferably from the range 0.1 - 0.5 wt.%.

12. A process according to claim 10, **characterized in that** the zeolite is a basic zeolite.

13. A process according to claim 10, **characterized in that** the zeolite consists of one or more representatives chosen from the groups of P-, A-, X- and Y-zeolites with the general formula
0.8-1.5 $Na_2O$. $Al_2O_3$. 0.8-24 $SiO_2$, where Si:Al = 1 for A- and P-zeolites and Si:Al = 1 - 6 for X- and Y-zeolites.

14. A process according to claim 1, **characterized in that** the glyceride oil to be modified consists of a mixture of

>   a. a palm oil fraction and palm kernel oil, or
>   b. a palm oil fraction and coconut oil or
>   c. soybean oil and fully hydrogenated soybean oil.

**Patentansprüche**

1.  Verfahren zur Ölmodifizierung, umfassend Aussetzen eines Glyceridöls einem Lipase-Katalysator mit der Wirkung zur Umlagerung von Fettsäureresten an den Glyceridmolekülen, wobei die Umlagerung unter Verwendung eines Lipase-Katalysators, der einen Produktivitätswert (LP) > 2500 hat - was bedeutet, dass in einem Zeitraum von drei Wochen mindestens 2500 kg Glyceridöl enzymatiisch bei einem Verbrauch von 1 kg Lipase-Katalysator zu einem Grad von 95 % umgelagert werden - zu einem Grad von 5 bis 99 % durchgeführt wird, wobei das Verfahren mit einem industriellen Durchsatz von mindestens 4000 kg Öl in den ersten 48 Stunden abläuft und wobei das Öl in einer Vorbehandlung einem Adsorbens ausgesetzt wird, welches in dem Öl unter Bildung einer Öl/Adsorbens-Dispersion dispergiert wird, und wobei das Adsorbens entfernt wird, bevor das Öl mit dem Lipase-Katalysator in Kontakt gebracht wird, und wobei die Öl/Adsorbens-Dispersion einer Scherenergie ausgesetzt wird, die in die Dispersion abgeführt wird und die 0,5 W/kg übersteigt, vorzugsweise 0,75 W/kg übersteigt, bevorzugter 1 W/kg übersteigt und noch weiter bevorzugt 2,5 W/kg übersteigt.

2.  Verfahren nach Anspruch 1, **dadurch gekennzeichnet, dass** die Lipase des Lipase-Katalysators an einer Trägersubstanz, vorzugsweise an Polypropylen oder Siliciumdioxid oder einem Gemisch dieser, immobilisiert wird.

3.  Verfahren nach Anspruch 1, **dadurch gekennzeichnet, dass** der Lipase-Katalysator Thermomyces lanuginosa-Lipase-Enzym enthält.

4.  Verfahren nach Anspruch 1, **gekennzeichnet durch** die Verwendung eines Festbettreaktors.

5.  Verfahren nach Anspruch 1, **dadurch gekennzeichnet, dass** das Öl während dem Ausgesetztseins der Lipase eine Temperatur hat, die im Bereich zwischen 30 - 75 °C, vorzugsweise im Bereich zwischen 60 - 70 °C, liegt.

6.  Verfahren nach Anspruch 1, **dadurch gekennzeichnet, dass** das Öl für einen Zeitraum, der aus dem Bereich 5 bis 12 Stunden und vorzugsweise aus dem Bereich 1 bis 6 Stunden ausgewählt ist, mit dem Adsorbens in Kontakt ist.

7.  Verfahren nach Anspruch 1, **dadurch gekennzeichnet, dass** das Öl während dem dem Adsorbens-Ausgesetztseins eine Temperatur hat, die im Bereich 30 - 150 °C, vorzugsweise im Bereich 40 - 110 °C und bevorzugter im Bereich 70 - 90 °C, liegt.

8.  Verfahren nach Anspruch 1, **dadurch gekennzeichnet, dass** das Öl vor dem dem Adsorbens-Ausgesetztseins einer üblichen Bleichbehandlung bei einer Temperatur, ausgewählt aus dem Bereich 85 - 220 °C, vorzugsweise aus dem Bereich 85 - 120 °C, unterworfen wird.

9.  Verfahren nach Anspruch 1, **dadurch gekennzeichnet, dass** das Adsorbens mit Hilfe eines Filtrationshilfsmittels, welches vorzugsweise Celite ist, aus dem Öl entfernt wird.

10. Verfahren nach Anspruch 1, **dadurch gekennzeichnet, dass** das Adsorbens ein Zeolith ist.

11. Verfahren nach Anspruch 10, **dadurch gekennzeichnet, dass** die Menge an Zeolith aus dem Bereich 0,01 - 10 Gew.-%, bezogen auf Öl, vorzugsweise aus dem Bereich 0,1 - 0,5 Gew.-%, ausgewählt wird.

12. Verfahren nach Anspruch 10, **dadurch gekennzeichnet, dass** der Zeolith ein basischer Zeolith ist.

13. Verfahren nach Anspruch 10, **dadurch gekennzeichnet, dass** der Zeolith aus einem oder mehreren Vertretern besteht, ausgewählt aus den Gruppen der P-, A-, X- und Y-Zeolithe mit der allgemeinen Formel 0,8-1,5 $Na_2O \cdot Al_2O_3 \cdot 0,8$-24 $SiO_2$, worin Si:Al = 1 für A- und P-Zeolithe und Si:Al = 1 - 6 für X- und Y-Zeolithe.

14. Verfahren nach Anspruch 1, **dadurch gekennzeichnet, dass** das zu modifizierende Glyceridöl aus einem Gemisch aus

    a. einer Palmölfraktion und Palmkernöl, oder
    b. einer Palmölfraktion und Kokosnussöl, oder
    c. Sojabohnenöl und vollständig hydriertem Sojabohnenöl

    besteht.


**Revendications**

1.  Procédé de modification d'une huile comprenant l'exposition d'une huile glycéride à une lipase servant de catalyseur ce qui provoque le réarrangement des résidus d'acide gras sur les molécules glycérides, lequel réarrangement est réalisé à un degré de 5 à 99 %, en utilisant un catalyseur lipase possédant une valeur de productivité (LP) supérieure à 2500 - ce qui signifie que sur une période de 3 semaines au moins 2500 kg d'huile glycéride sont réarrangés enzymatiquement jusqu'à un degré de 95 % avec une consommation de 1 kg de catalyseur lipase-lequel procédé est mis en oeuvre à un débit industriel d'au moins 4000 kg d'huile dans les premières 48 heures, et dans lequel l'huile dans un pré-traitement est exposée à un absorbant qui est dispersé dans l'huile pour former une dispersion huile/absorbant, et ledit absorbant est éliminé avant que l'huile ne soit mise en contact avec le catalyseur lipase, et dans lequel la dispersion d'huile/absorbant est exposée à une énergie de cisaillement dissipée dans la dispersion qui excède 0,5 W/kg, de préférence qui excède 0,75 W/kg, plus préférablement qui excède 1 W/kg et encore plus préférablement qui excède 2,5 W/kg.

2.  Procédé selon la revendication 1, **caractérisé en ce que** la lipase du catalyseur lipase est immobilisée sur une substance support, de préférence sur un polypropylène ou une silice ou un mélange de ceux-ci.

3.  Procédé selon la revendication 1, **caractérisé en ce que** le catalyseur lipase contient l'enzyme lipase de *Thermomyces lanuginosa*.

4.  Procédé selon la revendication 1, **caractérisé par** l'utilisation d'un réacteur à lit à garnissage.

5.  Procédé selon la revendication 1, **caractérisé en ce que** pendant l'exposition à la lipase, l'huile a une température comprise dans la plage allant de 30 à 75°C, de préférence dans la plage allant de 60 à 70°C.

6.  Procédé selon la revendication 1, **caractérisé en ce que** l'huile est en contact avec l'absorbant pendant une période choisie comprise dans la plage allant de 5 minutes à 12 heures et de préférence dans la plage allant de 1 à 6 heures.

7. Procédé selon la revendication 1, **caractérisé en ce que** pendant l'exposition à l'absorbant, l'huile a une température comprise dans la plage allant de 30 à 150°C, de préférence dans la plage allant de 40 à 110°C et plus préférablement dans la plage allant de 70 à 90°C.

8. Procédé selon la revendication 1, **caractérisé en ce que** l'huile avant l'exposition à l'absorbant est soumise à un traitement blanchissant habituel à une température choisie dans la plage allant de 85 à 220°C, de préférence dans la plage allant de 85 à 120°C.

9. Procédé selon la revendication 1, **caractérisé en ce que** l'absorbant est éliminé de l'huile au moyen d'un agent de filtration, qui est de préférence la célite.

10. Procédé selon la revendication 1, **caractérisé en ce que** l'absorbant est une zéolite.

11. Procédé selon la revendication 10, **caractérisé en ce que** la quantité de zéolite est choisie dans la plage allant de 0,01 à 10 % en poids par rapport à l'huile, de préférence dans la plage allant de 0,1 à 0,5 % en poids.

12. Procédé selon la revendication 10, **caractérisé en ce que** la zéolite est une zéolite basique.

13. Procédé selon la revendication 10, **caractérisé en ce que** la zéolite est constituée d'un ou de plusieurs composés représentatifs choisis dans le groupe de P-, A-, X- et Y-zéolites ayant la formule générale 0,8-1,5 $Na_2O$ . $Al_2O_3$ . 0,8-24 $SiO_2$, où Si/Al = 1 pour A- et P-zéolites et Si/Al = 1-6 pour X- et Z-zéolites.

14. Procédé selon la revendication 1, **caractérisé en ce que** l'huile glycéride devant être modifiée est constituée d'un mélange de

    a. un fraction d'huile de palme et de l'huile de palmiste, ou
    b. une fraction d'huile de palme et de l'huile de coco, ou
    c. de l'huile de soja et de l'huile de soja totalement hydrogénée.